# EUROPEAN PATENT APPLICATION

(11) **EP 3 834 831 A1**
(43) Date of publication of application: **16.06.2021**
(21) Application number: 19215524.0
(22) Date of filing: 12.12.2019
(51) Int. Cl.: A61K 31/727, A61K 31/37, A61K 31/4439, A61K 31/444, A61K 31/4545, A61K 31/5377, A61K 31/7024, A61P 9/14

(54) **TREATMENT OF HEMORRHOIDS WITH ANTICOAGULANT DRUGS**

(71) Applicant: de Keulenaer, Hendrik, 9120 Melsele (BE)
(72) Inventor: de Keulenaer, Hendrik, 9120 Melsele (BE)
(74) Representative: Algemeen Octrooi- en Merkenbureau B.V.

(57) **Abstract**

The present invention relates to an anticoagulant drug for use in the treatment of hemorrhoids. The anticoagulant drug is selected from the group consisting of heparins, factor Xa inhibitors, Direct Oral Anticoagulants, Vitamin K antagonists, bivalirudin, protein C, antithrombin III, thromolytics, thrombocyte aggregation inhibitors and combinations thereof. The treatment comprises administering the anticoagulant drug daily for a period from 3 to 60 days.

## Description

The present invention relates to the treatment of hemorrhoids with anticoagulant drugs.

Hemorrhoids, also called piles, can develop inside the rectum (internal hemorrhoids) or under the skin around the anus (external hemorrhoids).

Hemorrhoids are described as the abnormal downward displacement of the anal cushions causing venous dilatation. On histopathological examination, changes seen in the anal cushions include abnormal venous dilatation, vascular thrombosis, degenerative processes in the collagen fibers and fibroelastic tissues, and distortion and rupture of the anal subepithelial muscle . In severe cases, a prominent inflammatory reaction involving the vascular wall and surrounding connective tissue has been associated with mucosal ulceration, ischemia, and thrombosis (Zhifei Sun,Md and John Migaly, MD; Clin. colon rectal surg. 2016, March; 29(1):22-29 Review of Hemorrhoid Disease: presentation and Management).

Due to the study of Goenka et al (Goenka MK, Kochhar R, Nagi B, Mehta SK (1991) Rectosigmoid varices and other mucosal changes in patients with portal hypertension. Am J Gastroenterol 86:1185-1189) in which patients with portal hypertension and varices do not have an increased incidence of hemorrhoids, the theories of hemorrhoids as anorectal varices are now obsolete. Nevertheless the exact pathophysiology of symptomatic hemorrhoid disease is poorly understood.

Still, hemorrhoid disease is the fourth leading outpatient gastrointestinal diagnosis with an incidence of 5% of the population mainly between 45-65 years. The symptoms caused can greatly affect quality of life of the patients. Current treatment options comprise mostly advice on lifestyle changes, e.g. diet or exercise. A further treatment option is rubber band ligation or surgical intervention. However, there is still a need for a better treatment solution.

The present invention thus provides an anticoagulant drug for use in the treatment of hemorrhoids.

Anticoagulant drugs are drugs that prevent or reduce coagulation of blood, prolonging clotting time. According to the invention, the anticoagulant drug is selected from the group consisting of heparins, factor Xa inhibitors, Direct Oral Anticoagulants, Vitamin K antagonists, bivalirudin, protein C, antithrombin III, thromolytics, thrombocyte aggregation inhibitors and combinations thereof.

Preferably, the anticoagulant drug is selected from heparins, factor Xa inhibitors, Direct Oral Anticoagulants, Vitamin K antagonists and combinations thereof.

The dose of the anticoagulant drug to be used in the invention is the daily dose as recommended for the treatment of deep vein thrombosis with the anticoagulant drug. Such daily dose can be easily retrieved by the person skilled in the art, for instance from a medical compendium or from the Summary of Product Characteristics (SmPC).

The treatment according to the invention is from several days to several weeks, depending on the outcome of the treatment. The outcome of the treatment can be determined by observation of the hemorrhoids and/or feedback from the patient. Preferably, the treatment the treatment comprises administering the anticoagulant drug daily for a period from 3 to 60 days, more preferably from 3 to 30 days.

The anticoagulant drug can be administered orally or by injection, depending on the type of anticoagulant drug used.

As described above, hemorrhoids are well defined structures and include pathologic or symptomatic hemorrhoids. According to their location they can be divided into internal hemorrhoids, located in the upper part of the anal canal (above a mucosal fold called the dentate line), and external, located below.

Internal hemorrhoids are characterized by bleeding and varying degrees of prolapse, and may be followed by itching, pain, mucus discharge, and fecal seepage. External hemorrhoids usually only give major symptoms when they thrombose and are then very painful. The invention relates to the treatment of both internal and external hemorrhoids.

There are several classes of anticoagulant drugs that can be used in the present invention which are described in detail below.

### Group 1

### Group 1A

According to an embodiment, the anticoagulant drug is selected from the group of heparins. Heparin is a glycosaminoglycan whose major anticoagulant effect is accounted for by a pentasaccharide with a high affinity for antithrombin III. The group of heparins includes unfractionated heparin (UFH), heparinoids such as danaparoid and fractions of heparin such as low molecular weight heparin (LMWH), for instance having a molecular weight 4000-6000 Da. In particular, the anticoagulant drug is selected the group of dalteparin, nadroparin, tinzaparin and enoxaparin.

Danaparoid sodium, is a non-heparin mixture of low molecular weight sulphated glycosaminoglycuronans derived from animal mucosa, comprising heparan sulphate, dermatan sulphate and a minor amount of chondroitin sulphates. Danaparoid sodium is administered by subcutaneous injection at a dose of 750 anti-factor Xa units, twice daily.

Dalteparin sodium (Fragmin) is a low molecular weight heparin fraction (mean molecular weight 6000 Daltons) produced from porcine-derived heparin sodium. It acts mainly through its ability to potentiate the inhibition of Factor Xa and thrombin by antithrombin.

The preferred daily dosage for dalteparin is 150-200 IU/kg total body weight. Dalteparin sodium is administered once daily by subcutaneous injection.

Nadroparin calcium (Fraxiparin or Fraxodi) is a low molecular weight heparin fraction (mean molecular weight of 4500 Daltons) derived from porcine sources. It inhibits the activation of thrombin (factor Ila) by factor Xa.

The preferred daily dosage for nadroparin is 150-200 IU/kg total body weight. Nadroparin is administered twice daily by subcutaneous injection.

Tinzaparin sodium (Innohep) is a low molecular weight heparin fraction (mean molecular weight of 6000 Daltons) obtained from depolymerization of heparin from porcine intestinal mucosa. It acts as a potent co-inhibitor of several activated coagulation factors, especially Factors Xa and Ila (thrombin).

The preferred daily dosage for tinzaparin is 150-200 IU/kg total body weight, administered once daily by subcutaneous injection.

Enoxaparin sodium (Clexane) is a low molecular weight heparin fraction with a mean molecular weight of 4,500 daltons. In enoxaparin the antithrombotic and anticoagulant activities of standard heparin have been dissociated. Enoxaparin sodium has a high anti-Xa activity and low anti-Ila or anti thrombin activity.

The preferred daily dosage for enoxaparin is 100 IU/kg total body weight, administered once daily by subcutaneous injection.

### Group 1B

According to an embodiment, the anticoagulant drug is bivalirudin. Bilvarudin (Angiomax) is a direct thrombin inhibitor, which is a synthetic 20 amino acid peptide.

### Group 1C

According to an embodiment, the anticoagulant drug is selected from the group of Factor Xa inhibitors. Factor Xa inhibitors are a type of anticoagulant that work by selectively and reversibly blocking the activity of clotting factor Xa, preventing clot formation. In particular, the anticoagulant drug is fondaparinux (Arixtra).

Fondaparinux sodium is a synthetic and selective inhibitor of activated Factor X (Xa). The antithrombotic activity of fondaparinux is the result of antithrombin III (ATIII) mediated selective inhibition of Factor Xa. By binding selectively to ATIII, fondaparinux potentiates the innate neutralization of Factor Xa by ATIII.

The preferred dosage for fondaparinux is 7.5 mg once a day for a subject with a weight of 50 to 100 kg by subcutaneous injection.

### Group 1D

According to an embodiment, the anticoagulant drug is Protein C. Protein C (Ceprotin) is purified protein C from human plasma.

### Group 1E

According to an embodiment, the anticoagulant drug is Antithrombin, in particular antithrombin III.

### Group 2

According to an embodiment, the anticoagulant drug is selected from the group of Direct Oral Anticoagulants (DOACs). Contrary to the heparins described above, DOAC's can be administered orally. They are sometimes also referred to as non-vitamin K oral anticoagulants. They directly inhibit specific proteins within the coagulation cascade, whereas Vitamin K antagonists inhibit the synthesis of vitamin K dependent clotting factors. In particular, the anticoagulant drug is selected from the group of apixaban, dabigatran, edoxaban and rirvaroxaban.

Apixaban (Eliquis) is a potent, oral, reversible, direct and highly selective active site inhibitor of factor Xa. It does not require antithrombin III for antithrombotic activity. Apixaban inhibits free and clot-bound factor Xa, and prothrombinase activity. Apixaban has no direct effects on platelet aggregation, but indirectly inhibits platelet aggregation induced by thrombin. By inhibiting factor Xa, apixaban prevents thrombin generation and thrombus development. The dose of apixaban is 2.5-10 mg taken orally twice daily.

Dabigatran etexilate (Pradaxa) is a small molecule prodrug. After oral administration, dabigatran etexilate is rapidly absorbed and converted to dabigatran by esterase-catalysed hydrolysis in plasma and in the liver. Dabigatran is a potent, competitive, reversible direct thrombin inhibitor and is the main active principle in plasma. The dose of dabigatran is 150 mg taken orally twice daily (300 mg daily).

Edoxaban tosilate (Lixiana) is a highly selective, direct and reversible inhibitor of factor Xa, the serine protease located in the final common pathway of the coagulation cascade. Edoxaban inhibits free factor Xa, and prothrombinase activity. The oral dose of edoxaban is 60 mg once daily.

Rivaroxaban (Xarelto) is a highly selective direct factor Xa inhibitor with oral bioavailability. Inhibition of factor Xa interrupts the intrinsic and extrinsic pathway of the blood coagulation cascade, inhibiting both thrombin formation and development of thrombi. Rivaroxaban does not inhibit thrombin (activated factor II). Daily dose of rivaroxaban is from 10 to 30 mg.

### Group 3

According to an embodiment, the anticoagulant drug is selected from the group of Vitamin K antagonists. Vitamin K antagonists are also sometimes referred to as coumarins. Vitamin K antagonists exhibit their anticoagulant effect by inhibition of vitamin K epoxide reductase with a subsequent reduction of the gamma-carboxylation of certain glutamic acid molecules which are located at several sites near the terminal end both of coagulation factors II (prothrombin), VII, IX, and X and of protein C or its cofactor protein S.

Preferred Vitamin K antagonists for use in the present invention are phenprocoumon, acenocoumarol and warfarin. Phenprocoumon (Marcoumar) is also known as 4-Hydroxy-3-(1-phenylpropyl)-2H-chromen-2-one. Daily oral dose is from 3 to 12 mg.

Acenocoumarol (Sinthrome or Sintrom) is also known as 4-hydroxy-3-[1-(4-nitrophenyl)-3-oxobutyl]chromen-2-one. Daily oral dose is from 1 to 4 mg.

Warfarin (Marevan) is also known as 4-hydroxy-3-(3-oxo-1-phenylbutyl)chromen-2-one. Daily oral dose is from 2 to 10 mg.

### Group 4

According to an embodiment, the anticoagulant drug is selected from thrombolytics, in particular alteplase, tenecteplase and urokinase, optionally in combination with heparin.

### Group 5

Other anticoagulant drugs that can be used according to the invention are for instance antibodies such as caplacizumab.

### Group 6

Other anticoagulant drugs that can be used according to the invention are thrombocyte aggregation inhibitors such as aspirin, clopidogrel, prasugrel, ticagrelor and GpIIb/IIIa inhibitors such as abciximab, eptifibatide and tirofiban.

### Examples

### Example 1

A 50 year old female suffering from hemorrhoid disease since 20 years presenting herself with a hemorrhoid thrombosis was treated with dalteparin with a dosage of 15000 IU. After 3 day therapy a subjective lowering of the pressure was experienced. The hemorrhoid thrombosis was completely cured after 14 days of therapy.The therapy was continued for a total of 30 days.

### Example 2

A 47 year old male truck driver presenting hemorrhoid thrombosis was treated with nadroparine (Fraxodi) with a dosage of 15200 IU aXa. The patient indicated a subjective better feeling after 4 days and after 15 days stopped his therapy because pain had disappeared.

### Example 3

A 64 year old male accountant presenting hemorrhoid thrombosis and prolapse was treated with tinzaparine (Innohep) with a dosage of 18000 IU. The patient indicated feeling better after 7 days of therapy. The therapy was continued for two months after which the patient was free of symptoms.

### Example 4

A 74 year old female (5 children + muscle laxity), suffering from hemorrhoid thrombosis and prolapse was treated with enoxaprine (Clexane) with a dosage of 80 mg, twice daily . The patient was free of symptoms after 6 days of therapy. Therapy was continued thereafter for 40 days.

### Example 5

A 45 year old female teacher with 3 children suffering from prolapse and hemorrhoid thrombosis was treated with fondaparinux (Arixtra) 7.5 mg daily subcutaneous injection. Symptoms cleared out after 8 days. Therapy was continued for 21 days .

### Example 6

A 54 year old male with an office job suffering from two hemorrhoid thrombosis was treated with apixaban (Eliquis) with a dosage of 10 mg twice daily for 7 days followed by a dosage of 5 mg twice daily. After 3 days there was relief of the complaints and symptoms cleared out after 9 days. Therapy was continued for 28 days.

### Example 7

A 48 year old female doing housekeeping suffering from one big hemorrhoid thrombosis was treated with dabigatran (Pradaxa) with a dosage of 150 mg twice daily. After 4 days of therapy there was relief of symptoms. Symptoms cleared after 8 days. Therapy was continued for 56 days.

### Example 8

A 71 year old female leading a sedentary life suffering from 2 hemorrhoid thrombosis was treated with edoxaban (Lixiana) with a dosage of 60 mg once daily. After 5 days of therapy the patient experienced relief of symptoms and after 12 days symptoms cleared.

### Example 9

A 48 year old male , CFO from a large company, working 12 hours a day mostly sitting after a desk, having 3 hemorrhoid thrombosis was treated with rivaroxaban(Xarelto) with a dosage of 15 mg twice daily for 3 weeks, followed by a dosage of 20 mg once daily. Symptoms cleared up after 3 days and complete relief of symptoms occurred after one week. Therapy was continued for 2 months.

### Example 10

A 58 year old male engineer suffering from 2 hemorrhoid thrombosis was treated with phenprocoumon (Marcoumar) with a therapeutic dosage with INR 2-3. After reaching an INR between 2-3, the hemorrhoids cleared up very quickly (4 days).

### Example 11

A 51 year old female teacher suffering from 1 hemorrhoid thrombosis was treated with acenocoumarol (Sintrom) with a therapeutic dosage with INR 2-3. After reaching a therapeutic INR , the thrombosis cured after 3 days.

### Example 12

A 64 year retired construction worker was treated with warfarin (Marevan) with a therapeutic dosage with INR 2-3. After reaching a therapeutic INR 2-3 , the thrombosis of two hemorrhoids cleared up very quickly in 5 days

## Claims

1. Anticoagulant drug for use in the treatment of hemorrhoids.

2. Anticoagulant drug according to claim 1, selected from the group consisting of heparins, factor Xa inhibitors, Direct Oral Anticoagulants, Vitamin K antagonists, bivalirudin, protein C, antithrombin III, thromolytics, thrombocyte aggregation inhibitors and combinations thereof.

3. Anticoagulant drug according to claim 2, selected from the group consisting of heparins, factor Xa inhibitors, Direct Oral Anticoagulants, Vitamin K antagonists and combinations thereof.

4. Anticoagulant drug according to any one of the preceding claims, wherein the anticoagulant drug is selected from the group of heparins, preferably unfractionated heparin, heparinoids and low molecular weight heparins, preferably dalteparin, nadroparin, tinzaparin and enoxaparin.

5. Anticoagulant drug according to any one of claims 1 to 3, wherein the anticoagulant drug is selected from the group of factor Xa inhibitors, and wherein the anticoagulant drug is preferably fondaparinux.

6. Anticoagulant drug according to any one of claims 1 to 3, wherein the anticoagulant drug is selected from the group of Direct Oral Anticoagulants, preferably apixaban, dabigatran, edoxaban and rivaroxaban.

7. Anticoagulant drug according to any one of claims 1 to 3, wherein the anticoagulant drug is selected from the group of Vitamin K antagonists, preferably phenprocoumon, acenocoumarol and warfarin.

8. Anticoagulant drug according to any one of the preceding claims, wherein the treatment comprises administering the anticoagulant drug daily for a period from 3 to 60 days.

9. Anticoagulant drug according to any one of the preceding claims, wherein the hemorrhoids include internal and external hemorrhoids.
